(19) Europäisches Patentamt
European Patent Office
Office européen des brevets

(11) Publication number: **0 152 275 B1**

(12) # EUROPEAN PATENT SPECIFICATION

(45) Date of publication of patent specification: **19.06.91**

(51) Int. Cl.[5]: **C12N 9/10**, C12N 15/54, C12P 13/04, C12P 13/22

(21) Application number: **85300802.7**

(22) Date of filing: **06.02.85**

(54) **Production of amino acids via bioconversion.**

(30) Priority: **07.02.84 GB 8403244**

(43) Date of publication of application:
**21.08.85 Bulletin 85/34**

(45) Publication of the grant of the patent:
**19.06.91 Bulletin 91/25**

(84) Designated Contracting States:
**DE FR GB IT**

(56) References cited:
**EP-A- 0 116 860**

**CHEMICAL ABSTRACTS, vol. 83, no. 5, 4th August 1975, page 194, abstract no. 39398z, Columbus, Ohio, US; C. MAVRIDES et al.: "Multispecific aspartate and aromatic amino acid aminotransferases in Escherichia coli", & J. BIOL. CHEM. 1975, 250(11), 4128-33**

(73) Proprietor: **THE NUTRASWEET COMPANY (a Delaware corporation)**
**1751 Lake Cook Road**
**Deerfield Illinois 60015(US)**

(72) Inventor: **Lewis, David John**
**Wilden Kiln Road**
**Prestwood, Bucks(GB)**
Inventor: **Farrand, Stephen Giles**
**1, Glynswood House Greenwood Meadow**
**Chinnor Oxon(GB)**

(74) Representative: **Goldin, Douglas Michael et al**
**J.A. KEMP & CO. 14, South Square Gray's Inn**
**London WC1R 5EU(GB)**

**EP 0 152 275 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

INTRODUCTION

The production of amino acids in accordance with a transamination reaction involves the covalent transfer of an amino group from an amine donor to an acceptor alpha-keto-acid precursor of the amino acid in the presence of an aminotransferase. A transamination is illustrated by the following general reaction:

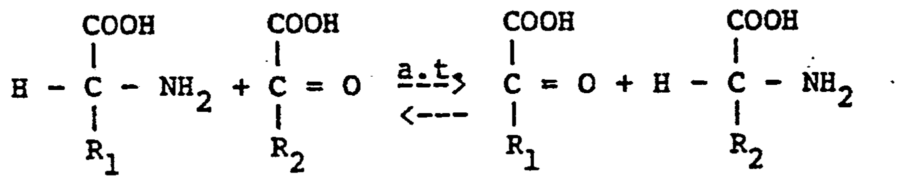


(I) (II) (III) (IV)

wherein (I) is an amine donor; (II) is a keto acid precursor, (III) is a keto acid product and (IV) is an amino acid and a.t. is an aminotransferase. The reaction is reversible, thus the aminotransferase may be utilized both in the synthesis and the degradation of an amino acid.

Amino acid synthesis often involves transamination as the final step in biosynthesis. For example, the biosynthesis of the aromatic amino acids phenylalanine and tyrosine requires the action of an aminotransferase as the final step. Phenylpyruvic acid is converted to phenylalanine by an aminotransferase that transfers an amino group from glutamate. The keto-acid product of this reaction is$\alpha$-ketoglutaric acid. Similarly, an amino group from glutamate is transferred to 4-hydroxyphenylpyruvic acid to produce tyrosine.

The aminotransferases employed in the transamination reactions may be obtained from a variety of sources, in particular, from microorganisms producing the aminotransferase. Naturally occurring microorganisms, so-called "wild-type" microorganisms, are capable of producing a variety of aminotransferases.

In general, the rate of amino acid production in accordance with a transamination reaction is determined by the activity of the particular aminotransferase employed and the temperature at which the transamination reaction is conducted. Most aminotransferases produced by wild-type microorganisms lose significant activity at temperatures equal to or greater than 4Ø° C. In addition, the aminotransferases produced by wild-type microorganisms lose activity in the presence of high concentrations of a keto acid precursor, and/or amino acid product.

It is an object of the present invention to provide a method to increase the rate of amino acid production in accordance with a transamination reaction by reducing the effects of the loss of aminotransferase activity due to temperature and to enable one to conduct a transamination reaction using high concentrations of keto acid precursor.

In addition, it is an object of the present invention to provide a method for producing amino acids in accordance with transamination reaction using an aminotransferase obtained from a genetically modified microorganism capable of reproducing the aminotransferase wherein the microorganism is made permeable during the transamination reaction.

It is a further object of the present invention to provide a process for producing amino acids in accordance with a transamination reaction conducted at a temperature greater than the temperature at which the aminotransferase will ordinarily limit the usefulness of the process.

SUMMARY OF THE INVENTION

The present invention provides a process for preparing an amino acid by transamination between an amine donor and keto acid precursor of the amino acid in the presence of an aminotransferase obtained from a microorganism which has been genetically modified to overproduce the aminotransferase wherein the transamination is carried out at a temperature equal to or greater than 40° C.

The present invention also provides a process for preparing phenylalanine by transamination between an amine donor and phenylpyruvic acid in the presence of an aminotransferase obtained from a microorganism which has been genetically modified to overproduce the aminotransferase, wherein the keto acid precursor concentration is greater than 10g/L.

DETAILED DESCRIPTION OF THE INVENTION

In accordance with the process of the present invention, high yields of an amino acid are obtained at a high rate of production utilizing a transamination reaction employing a genetically modified microorganism that overproduces the aminotransferase. The term "genetically modified microorganism that overproduces the aminotransferase" refers to a genetically modified microorganism that produces the aminotransferase at a level of at least 200U/g dry weight cells. As used herein, the term "rate of conversion" refers to the "grams of amino acid produced/gram dry weight of cells (or microorganisms) present X time to equilibrium" or to the grams of amino acid produced/$10^4$ Units of enqyme activity X time to equilibrium. As used herein, the term "Units of enzyme activity" refers to the enzymatic activity of the reaction mixture produced by the microorganisms present in the reaction mixture. The term "time to equilibrium" refers to the time at which the maximum concentration of amino acid is produced by the reaction.

In accordance with a preferred embodiment of the present invention a microorganism capable of overproducing aminotransferase of interest is grown in suitable media. The microorganism generally contains a plasmid carrying a gene encoding an aminotransferase capable of transaminating a keto acid precursor of the amino acid to be produced. Such media are well recognized by one of ordinary skill in the art and include for example, a media comprising yeast extract, glutamate and mineral salts. A preferred media comprises glucose and mineral salts. Such media are generally commercially available or readily prepared by one of ordinary skilled in the art. The cells are cultivated within a temperature range of from 25° to 4∅° C and preferably about 37° C for approximately sixteen hours or until the concentration of cells is preferably at least 1∅g/L and most preferably 3∅g/L. The cells may be utilized in the presence of the growth media or harvested by centrifugation. In addition the cells may be utilized intact or may be treated to release the aminotransferase. Techniques for releasing the aminotransferase are well known in the art and include, for example, treatment of the cell with a surfactant, e.g., 0.2% Triton X-1∅∅ ∅.2% deoxycholate etc.; physical breakage via freeze/thawing; sonication; pressure disruption and the like. It has been surprisingly found that if intact cells are utlized in accordance with the procedure of the present invention, the cells are made permeable at temperatures at which the transamination reaction is conducted.
Specifically, it has been found that when the process of the present invention is conducted at temperatures greater than 4∅° C, the cells are permeabilized. In an embodiment of the present invention it is preferred not to permeabilize the cells prior to the transamination.

Therefore intact cells may be added to a mixture containing the amine donor and keto acid precursor. If the cells are permeabilized prior to conducting the transamination, a portion of the solution containing the released aminotransferase is added to a mixture containing an amine donor and a keto acid precursor. Preferably the resulting reaction mixture is incubated for a period of time sufficient to allow the reaction mixture to reach equilibrium.

Depending on the specific aminotransferase employed, the pH of the reaction-mixture is maintained within a range of from 5 to 9. In the production of phenylalanine, the reaction mixture is preferably maintained within a range of from 6.5 to 9 and more preferably of from 7.5 to 8.5. Suitable buffers may be used to maintain the pH of the reaction mixture within a range sufficient to maintain enzyme activity.

In order to increase the yield of amino acid and stabilize the reaction mixture, a cofactor to the aminotransferase such as pyridoxal-5-phosphate, pyridoxine, pyridoxamine or derivatives thereof may be added to the reaction mixture. The quantity of cofactor to be added to the reaction mixture is readily ascertained by one of ordinary skill in the art.

Furthermore, the addition of a formic acid solution to the reaction mixture may increase the rate of reaction and production of the amino acid. It is preferred to employ a formic acid concentration in the range of from about ∅.5M to about 15M. The addition of formic acid to the reaction mixture may increase the rate of reaction by increasing the activity of the aminotransferase and stabilizing the aminotransferase particularly if the reaction is conducted at temperatures equal to or greater than 4∅° C.

Representative amino acids produced in accordance with the methods of the present invention include, but is not limited to, L-arginine, L-glutamine, L-serine, L-tyrosine, L-phenylalanine, L-tryptophane, L-dihydroxyphenylalanine (L-DOPA), L-histidine, L-lysine, L-isoleucine, L-methionine and the like. The methods of the present invention are particularly effective in producing aromatic amino acids such as for example L-phenylalanine, L-tyrosine, and the like.

The selection of the specific amine donors and keto acid precursors employed in the process of the present invention is dependent upon the specific amino acid to be produced and is readily ascertained by one of ordinary skill in the art. A wide variety of amine donors may be employed depending upon the aminotransferase utilized. If aspartate aminotransaminase is employed as the aminotransferase, glutamic acid, alanine, aspartic acid, tyrosine or derivatives thereof, may be employed as amine donors. The keto

acid precursor employed depends upon the specific amino acid to be prepared. The keto acid precusor may be utilized in the form of the free acid or salt thereof. For example, in the production of phenylalanine, phenylpyruvic acid or derivatives thereof, is employed as the keto acid precursor. The following table illustrates suitable keto acid precursors that may be employed to produce various amino acids:

| Amino Acid | Keto Acid Precursor |
|---|---|
| L-glutamine | 4-oxo-pentanedioic acid-1-amide |
| L-serine | hydroxypyruvic acid |
| L-tryptophan | indole-3-pyruvic acid |
| L-histidine | 4-imidazolepyruvic acid |
| L-isoleucine | 3-methyl-2-oxopentanoic acid |
| L-methionine | alpha-keto-gamma-methiol-butyric acid |
| L-tyrosine | p-hydroxyphenylpyruvic acid |
| L-dihydroxyphenylalanine | dihydroxyphenylpyruvic acid |

The ratio of amine donor to keto acid precursor may be readily ascertained by one of ordinary skill in the art. Although, the theoretical ratio of amine donor to keto acid precursor should be at least 1:1, it is preferred to employ an excess of the amine donor. For example, in the production of phenylalanine, it has been found that if glutamic acid is employed as the amine donor, a ratio of glutamic acid:phenylpyruvic acid in the range of from about 3:1 to 4:1 is preferred. Furthermore, it has been found that if aspartic acid is employed as the amine donor in the production of phenylalanine, it is preferred to employ a ratio of aspartic acid: phenylpyruvic acid in the range of from about 1:1 to 1.5:1 and most preferred in a range of 1:1 to 1.2:1.

It has been surprisingly found that keto acid precursor concentrations greater than 1Øg/L have been effective in the process of the present invention. In the production of L-phenylalanine, it has been found that concentrations of phenylpyruvic acid in the range of 3Ø-6Øg/L are preferred. In addition it has been found that reaction by-products produced in the preparation of phenylpyruvic acid and generally transferred to the transamination reaction mixture when phenylpyruvic acid is employed as the keto acid precursor, (i.e., salts, dimers, etc.) do not affect the yields of phenylalanine. Furthermore, it has been found that the phenylpyruvic acid employed as the keto acid precursor may be commercially purchased or prepared in situ and employed without isolation or purification in the transamination reaction of the present invention. Phenylpyruvic acid may be prepared by reacting benzaldehyde and hydantoin to yield benzylidenehydantoin and then hydrolyzing the benzylidenehydantoin to phenylpyruvic acid or salts thereof.

In addition to temperature and keto acid precursor concentration, the aminotransferase activity of a reaction mixture depends upon the amount of cells containing the aminotransferase added to the reaction mixture. The lower the aminotransferase activity of each individual cell, the more cells that must be added to the reaction mixture to sustain the reaction. However, the greater the amount of cells added to the reaction mixture, the greater the difficulty in isolating the amino acid product. One advantage associated with the process of the present invention in employing a genetically modified microorganism in lieu of a wild type microorganism is that it has been found that it is possible to maximize the aminotransferase activity in the reaction mixture and minimize the total amount of cells present, thereby facilitating product isolation. The low cell density of the reaction mixture in the process of the present invention enhances amino acid product recovery by facilitating the removal of cell debris. It has been found that a concentration of cells sufficient to yield an aminotransferase activity of o.5 x $1Ø^4$ U/L or greater is preferred when conducting the process of the present invention.

The amino acids produced in accordance with the methods of the present invention may be isolated in accordance with conventional techniques. For example, a water miscible solvent wherein the amino acid of interest is insoluble may be added to the reaction mixture. Upon addition of the water miscible solvent, the amino acid of interest will precipitate and thus be removed from the reaction mixture. This method for isolating the amino acid product may be of particular importance with respect to amino acids such as

phenylalanine, that inhibit the aminotransferase employed in the transamination reaction.

In accordance with the process of the present invention, a wide variety of aminotransferases produced by a microorganism may be effective in the procedures of the present invention. Examples of such aminotransferases include but are not limited to aminotransferases such as aspartate aminotransferases, branched chain aminotransferases and aromatic aminotransferases as defined by the gene products E.coli genes aspC, tyrB and ilyE and the like. The above-mentioned aminotransferases when produced using a genetically modified microorganism are not necessarily pure. However, it has been found that unpurified crude extracts containing the aminotransferase of interest may be employed in the process of the present invention. These extracts may be of course, partially purified prior to being utilized in accordance with the methods of the present invention. A mixture of two or more aminotransferases may also be employed in accordance with the methods of the present invention. In the preparation of phenylalanine in accordance with the methods of the present invention, aspartate aminotransferase is the preferred aminotransferase employed.

In view of the fact that production of amino acids by conventional transamination reactions is limited by aminotransferase activity and that increasing the temperature of the reaction substantially reduces and/or effectively inhibits aminotransferase activity, it has been found that in order to produce amino acids in accordance with a transamination reaction at temperatures equal to or greater than 40° C, it is necessary to increase the aminotransferase activity to a level sufficiently high to overcome effects of denaturation of the aminotransferase due to temperature and/or high keto acid concentration. Because a wild type microorganism will not produce aminotransferases in quantities sufficient to conduct the processes of the present invention, it is necessary to employ a genetically modified microorganism which will over-produce the aminotransferase. As readily ascertained by one of ordinary skill in the art, microorganisms may be readily genetically modified in accordance with conventional techniques. Such techniques include, for example, mutagenesis, transduction, transformation, (e.g. with multi copy plasmids), conjugation and in vitro gene manipulation. For example, aminotransferase over-producing strains may be isolated after mutagenesis. Alternatively, the gene for the appropriate aminotransferase may be cloned into a suitable plasmid or phage vector. A further embodiment invokes isolation of the aminotransferase gene on a suitable vector and the subsequent in vitro or in vivo manipulation so as to elevate expression of the aminotransferase gene. This may be accomplished for example, by fusing the gene to a more powerful constitutive promoter. Then, the isolated, mobilized aminotransferase gene may be inserted back into the microorganism chromosome by techniques such as recombination, transposition or phage interactions.

The microorganisms which may be genetically modified as described herein include but are not limited to, for example, Achromobacter aquatilis, Achromobacter liquidum, Aspergillus oryzae, Aspergillus niger, Bacillus species such as Bacillus megaterium, Bacillus subtilis, Bacterium succinium, Brevibacterium species such as Brevibacterium flavum, coryneforms including Corynebacterium species such as Corynebacterium glutaminicum, Erwinia herbicola, Escherichia coli, Gluconobacter melanogenus, Lactobacillus bulgaris, Micrococcus ureae, Penicillium vinaceum, Proteus vulgaris, Pseudomonas species such as Pseudomonas aeruginosa, Pseudomonas dacunhae, Pseudomonas putida, Sarcina lutea, Streptomyces griseus, Serratia marcescens, Streptomyces phaeochromogenus, and Saccharomyces species and Schizosaccharomyces species. These microorganisms may not necessarily be intact living cells, but may be lyophilized, heat-treated or treated with acetone and the like to permeabilize the cells prior to use thereof in the present invention.

For the purpose of illustrating the processes of the present invention, a strain rich in aminotransferase may be constructed by cloning the gene for the aspartate aminotransferase from E-coli K12 (aspC) into the multi-copy plasmid pAT153. The cloned strain is transformed into a suitable strain such as E.coli K12 W3110, and the resulting strain over-produces the aspC gene product from 10-100 times. The aspartate aminotransferase is known to be active in the transaminations; i.e., the conversion of phenylpyruvic acid to phenylalanine; using both L-aspartic acid and L-glutamic acid as amine donors. The aspC encoded protein has the additional benefit of being relatively heat stable.

An aspC clone may preferably be obtained by ligating suitably restricted DNA from either E.coli K12 or another suitable microorganism into a suitable vector such as pAT153. The resulting ligation reaction is then used to transform a suitable recipient such as HW159 (ATCC 39260) deposited in American Type Culture Collection, 12301 Parklawn Drive, Rockville, Maryland 20852 on 6 January 1983 under Budapest Treaty Conditions. This strain (HW159) lacks both the aspartate aminotransferase (aspC) and aromatic aminotransferase (tyrB) genes. The result of these two lesions is that the strain requires tyrosine, aspartate and to a lesser extent, phenylalanine for growth. Recombinants that carry aspC or tyrB clones may be readily identified by their ability to grow on minimal medium in the absence of tyrosine, aspartate and phenylalanine. AspC clones may be differentiated from tyrB clones by analysis of cells extracts by native

polyacrylamide gel electrophoresis followed by staining for phenylpyruvic acid aminotransferase activity. Isolation of an aspC clone may be further facilitated by the use of a specialised transducing phage such as Lambda aspC as a source of DNA in the original ligation.

The microorganism (ATCC 39501) used in the following examples was constructed by transforming an E.coli K12 prototroph with the plasmid pME98, a pAT153 derivative carrying the aspC gene on a 3.4kb fragment of DNA. ATCC 39501 was deposited in the American Type Culture Collection on 4th November 1983 under Budapest Treaty conditions. All the techniques are conventional techniques readily ascertained by one of ordinary skill in the art.

One embodiment of the present invention relates to an improved process for preparing an amino acid in accordance with a transamination reaction involving an amine donor and keto acid precursor of the amino acid in the presence of an aminotransferase obtained from a microorganism which has been genetically modified to overproduce the aminotransferase, wherein the improvement comprises conducting the transamination reaction at a temperature equal to or greater than 40°C. In the production of phenylalanine in accordance with the process of the present invention, it is preferred to conduct the transamination reaction within a temperature range of from 40°C - 70°C and most preferred to conduct the reaction within a temperature range of 45°C - 60°C.

Another embodiment of the present invention relates to an improved process for preparing phenylalanine in accordance with a transamination reaction involving an amine donor and keto acid precursor of phenylalanine in the presence of an aminotransferase obtained from a microorganism which has been genetically modified to overproduce the aminotransferase, wherein the improvement comprises having a keto acid precursor concentration of greater than 10 g/L. It is preferred to have a keto acid precursor concentration in a range of from 30 to 50 g/L and preferably from 40 to 50 g/L and to conduct the transamination reaction within a temperature range of 45° - 70°C, particularly from 45°C to 55°C.

Another embodiment of the present invention relates to an improved process for preparing an amino acid in accordance with a transamination reaction involving an amine donor and keto acid precursor of the amino acid in the presence of aminotransferase obtained from a microorganism which has been genetically modified to overproduce the aminotransferase wherein the improvement comprises permeabilizing the microorganism during the course of the transamination reaction by conducting the reaction at a temperature equal to or greater than 40°C.

A further preferred embodiment involves the production of aromatic amino acids in accordance with a transamination reaction employing an aminotransferase obtained from a microorganism which has been genetically modified to overproduce the aminotransferase, wherein the reaction is conducted at a temperature equal to or greater than 40°C.

The following Examples are intended to further illustrate the present invention and not to limit the invention in spirit or scope. In the Examples, all parts are parts by weight unless otherwise expressly set forth. The term "cells" as used herein in the following Examples refers to genetically modified cells containing elevated levels of the aminotransferase required to conduct the transamination reaction.

Example 1

Escherichia coli containing a plasmid carrying a gene encoding for aspartate aminotransferase was grown in one of the following media: - 1) Yeast extract (10 g.), $Na_2HPO_4$ (6 g.), $KH_2PO_4$ (3 g.), NaCl (0.5 g.), $NH_4Cl$ (1 g.), $CaCl_2 \cdot 6H_2O$ (0.02 g.), $MgSO_4 \cdot 7H_2O$ (0.25 g.), Carbenicillin (0.1 g.) and water to 1 litre; 2) glucose (35g), $KHPO_4$ (6.6g), $(NH_4)_2HPO_4$ (2.0g), $MgSO_4.7H_2O$ (2.25g), ammonium ferric citrate (0.11g), indole acrylic acid (2.0mg) and water to 1 liter. The cells were cultivated at 33°C for a period of time sufficient to ensure at least 1 g dry weight of cells per litre. The cells were harvested by centrifugation and the aspartate aminotransferase was released by freezing and thawing the resulting cells suspension containing approximately 1 g dry weight of cells per liter.

Example 2

A solution containing aspartate aminotransferase was produced as in Example 1 from a suspension containing approximately 2 g dry weight cells per liter. The solution was added to a mixture comprising 15 g sodium phenylpyruvate monohydrate per liter, 19 g sodium aspartate monohydrate per liter, 10 mg pyridoxal phosphate per liter adjusted to pH 8.5 with 10N sodium hydroxide. The resulting reaction mixture was incubated for 4 hours at 60°C to yield a mixture containing 10.2 g L-phenylalanine per liter.

Example 3

A flask was shaken at 5ø° C with the following reaction mixture: 3.88g aspartic acid, 5ml cell suspension containing ø.14 X 1ø$^4$U aspartate aminotransferase, ø.375mg pyridoxal phosphate and 51ml of a reaction mixture prepared by heating under reflux, with stirring, for 3.5 hours, 4-hydroxy-5-benzylidene hydantoin (8.5øg/41.67mmole) sodium hydroxide pellets (48.øg/1.2ømole) and water (24øml). The reaction mixture was incubated overnight at 5ø° C and the resulting precipitate was filtered, washed and identified as L-tyrosine by NMR, TLC, HPLC and optical rotation ($[\alpha]^{2ø}$ = - 1ø.33° (C = 4, 1N HCl))

Example 4

A series of transamination reactions were conducted under various conditions in accordance with the procedure of Example 2. The temperature at which the reaction is conducted, the initial phenylpyruvic acid concentration ([PPA]), amine donor, time to equilibrium, yields of phenylalanine ([L-Phe]), grams of cells % Conversion and rate of production of phenylalanine (g phenylalanine/g cells x time to equilibrium) (Rate) is represented in the following table:

TABLE 1

| Temp. oC | [PPA] g/L | Amine donor g/L | | Time to Equilibrium (hr) | [L-phe] (g/L) | g cells L | Conversion | Rate |
|---|---|---|---|---|---|---|---|---|
| 33 | 30 | 36.5 | aspartic acid | 6.25 | 23.9 | 2.0 | 95 | 2.0 |
| 33 | 30 | 80.6 | glutamic acid | 0.4 | 25.8 | 1.4 | 87 | 32.3 |
| 33 | 60 | 73.0 | aspartic acid | 4.0 | 58.0 | 2.0 | 95 | 7.3 |
| 33 | 60 | 161.3 | glutamic acid | 4.0 | 40.7 | 2.0 | 82 | 9.0 |
| 43 | 40 | 48.6 | aspartic acid | 1.5 | 39.5 | 2.0 | 94 | 13.2 |
| 60 | 40 | 48.6 | aspartic acid | 1.0 | 34.0 | 2.0 | 94 | 17.0 |
| 60 | 30 | 233.9 | glutamic acid | 0.17 | 27.2 | 2.0 | 94 | 71.0 |

Example 5

A series of transamination reactions were conducted under various conditions in accordance with the procedure of Example 2. The temperature at which the reaction is conducted, the initial phenylpyruvic acid

concentration ([PPA]), amine donor, yields of phenylalanine ([L-Phe]), % Conversion and Units enzyme activity ("Units") and rate of production of phenylalanine (g phenylalanine/ Units of enzyme activity x time to equilibrium) (Rate) is represented in the following table:

TABLE 2

| Temp. oC | [PPA] g/L | Amine donor g/L | Time to Equilibrium (hr) | [L-phe] (g/L) | L-phe (g/L) | $10^4$ Units (U/L) | Conversion | Rate |
|---|---|---|---|---|---|---|---|---|
| 40 | 40 | 48.6 aspartic acid | 5.0 | 7.2 | 36.2 | 2.8 | 98 | 2.6 |
| 40 | 40 | 42.2 aspartic acid | 10.0 | 3.7 | 37.0 | 2.8 | 97 | 1.3 |
| 40 | 55 | 58.0 aspartic acid | 11.0 | 4.3 | 47.7 | 2.8 | 97 | 1.5 |
| 40 | 30 | 36.5 aspartic acid | 6.5 | 4.1 | 26.5 | 2.8 | 96 | 1.5 |
| 30 | 40 | 38.9 aspartic acid | 15.5 | 2.1 | 32.5 | 2.5 | 92 | 0.8 |
| 50 | 40 | 48.6 aspartic acid | 3.0 | 8.8 | 35.3 | 2.7 | 95 | 3.3 |
| 50 | 40 | 32.4 aspartic acid | 3.5 | 11.5 | 34.5 | 3.4 | 94 | 3.4 |

Example 6

A series of transaminations were conducted in accordance with the procedure of Example 2 at a temperature of 5Ø°C using 4Ø g phenylpyruvic acid per liter, 48.6g/L of aspartic acid and varying amounts of cells in order to vary the aminotransferase activity. The results in Table 4 illustrate the aminotransferase activity, grams of cells employed, and the time needed for the reaction to reach equilibrium.

TABLE 3

| Aminotransferase activity (U/L) | g. cells/L | Time to equilibrium (hr) |
|---|---|---|
| 5,774 | 1.69 | 23 |
| 1Ø,6ØØ | 1.55 | 7 |
| 22,33Ø | 1.43 | 4.1 |
| 81,ØØØ | 2.25 | 2.Ø |

Example 7

A reaction vessel containing a 9.6L of a mixture comprising 585g sodium phenylpyruvate monohydrate and 324g aspartic acid was heated to 5Ø°C and the pH of the mixture was controlled at pH 8.Ø by the addition of either 35% $NH_4OH$ or 18% HCl as appropriate. To the reaction mixture was added 4ØØml of an homogenized cell suspension containing 3.4 x $1Ø^5$ U aspartate aminotransferase and 75mg pyridoxal phosphate. The resulting mixture was stirred for 3.5 hours to yield a solution containing 33.9g phenylalanine/L, at a rate of 7.1g L-phenylalanine/$1Ø^4$ Units.hr.

Example 8

A reaction vessel containing a 9.7L of a mixture comprising 585g sodium phenylpyruvate monohydrate and 486g aspartic acid was heated to 5Ø°C and the pH of the mixture was controlled at pH 8.Ø by the addition of 35% $NH_4OH$. To the reaction mixture was added 275ml homogenised cell suspension containing 2.8 x $1Ø^4$ U aspartate aminotransferase and 75mg pyridoxal phosphate. The resulting mixture was stirred for 3.6 hours to yield a solution containing 36.2 g phenylalanine/L, at a rate of 3.6g L-phenylalanine/$1Ø^4$ Units.hr.

Example 9

A flask was shaken at 5Ø°C with the following reaction mixture made up to 5Øml with water - 1.Ø5g 3-methoxy-4-hydroxyphenylpyruvic acid, 1.3Øg sodium aspartate monohydrate, Ø.375 pyridoxal phosphate and 5ml homogenised cell suspension. The pH of the reaction mixture was adjusted to 8.Ø with 1N NaOH. The resulting reaction mixture was incubated at 5Ø°C for 2.5 hours to yield or mixture containing 11.3g/L of 3-methoxy-4-hydroxyphenylalanine.

Example 1Ø

A reaction mixture containing 2.49g sodium phenylpyruvate monohydrate, 3.16g sodium aspartate monohydrate Ø.375mg pyridoxal phosphate and 2.5ml intact cells containing Ø.2 x $1Ø^4$U aminotransferase and 5Øml water was adjusted to pH 8.Ø with IN NaOH.

The reaction mixture was incubated for 2 hours at 5Ø°C to yield 32.Øg of L-phenylalanine per liter.

Although this invention has been described with respect to specific modification, the details thereof are

not to be construed as limitations, for it will be apparent that various equivalents, changes and modification may be resorted to without departing from the spirit and scope thereof and it is understood that such equivalent embodiments are intended to be included therein.

## Claims

1. A process for preparing an amino acid by transamination between an amine donor and keto acid precursor of the amino acid in the presence of an aminotransferase obtained from a microorganism which has been genetically modified to overproduce the aminotransferase wherein the transamination is carried out at a temperature equal to or greater than 40° C.

2. A process according to claim 1 wherein the amino acid is an aromatic amino acid.

3. A process according to claim 2 wherein the amino acid is phenylalanine, tyrosine or hydroxymethoxyphenylalanine.

4. A process according to claim 3, wherein the amino acid is phenylalanine.

5. A process according to claim 4 wherein the keto acid precursor is phenylpyruvic acid and the transamination reaction is conducted at 40° C to 70° C.

6. A process according to claim 5 wherein the concentration of phenylpyruvic acid is 30-60g/L.

7. A process according to claim 5 or 6 wherein the transamination is conducted at 45° C to 60° C.

8. A process according to any one of claims 5 to 7 wherein the gene coding for the aminotransferase is aspC, tyr B or ily E.

9. A process according to claim 8 wherein the microorganism is Escherichia coli.

10. A process according to claim 9 wherein the Escherichia coli is strain ATCC 39501.

11. A process according to claim 3 wherein the amino acid is 3-methoxy-4-hydroxyphenylalanine.

12. A process according to any one of the preceding claims wherein the amine donor is glutamic acid or aspartic acid.

13. A process according to claim 12 wherein the amine donor is aspartic acid.

14. A process according to any one of the preceding claims wherein intact cells containing the microorganism are utilized.

15. A process according to any one of the preceding claims wherein formic acid is added to the reaction.

16. A process for preparing phenylalanine by transamination between an amine donor and phenylpyruvic acid in the presence of an aminotransferase obtained from a microorganism which has been genetically modified to overproduce the aminotransferase, wherein the keto acid precursor concentration is greater than 10g/L.

17. A process according to claim 16 wherein the amine donor is glutamic acid or aspartic acid.

18. A process according to claim 16 or 17 wherein the reaction is conducted at a temperature within a range of from 45° C to 70° C.

19. A process according to any one of claims 16 to 18, wherein the precursor concentration is 40 to 50 g/l.

20. A process according to any one of claims 16 to 19, wherein the gene coding for the aminotransferase is aspC, tyrB or ilyE.

21. A process according to claim 20, wherein the microorganism is Escherichia coli ATCC 39501.

**Revendications**

1. Procédé de préparation d'un acide aminé par transamination entre un donneur d'amine et un céto-acide précurseur de l'acide aminé en présence d'une aminotransférase obtenue à partir d'un micro-organimse qui a été génétiquement modifié pour produire en grande quantité l'aminotransférase, dans lequel la transamination est effectuée à une température égale ou supérieur à 40° C.

2. Procédé selon la revendication 1, dans lequel l'acide aminé est un acide aminé aromatique.

3. Procédé selon la revendication 2, dans lequel l'acide aminé est la phénylalanine, la tyrosine ou l'hydroxyméthoxyphénylalanine.

4. Procédé selon la revendication 3, dans lequel l'acide aminé est la phénylalanine.

5. Procédé selon la revendication 4, dans lequel le précurseur céto-acide est l'acide phénylpyruvique et la réaction de transamination est effectuée à 40-70° C.

6. Procédé selon la revendication 5, dans lequel la concentration de l'acide phénylpyruvique est de 30-60g/l.

7. Procédé selon la revendication 5 ou 6, dans lequel la transamination est effectuée à 45-60° C.

8. Procédé selon l'une quelconque des revendications 5 à 7, dans lequel le gène codant pour l'aminotransférase est aspC, tyrB ou ilyE.

9. Procédé selon la revendication 8, dans lequel le micro-organisme est Escherichia coli.

10. Procédé selon la revendication 9, dans lequel l'Escherichia coli est la souche ATCC 39501.

11. Procédé selon la revendication 3, dans lequel l'acide aminé est la 3-méthoxy-4-hydroxyphénylalanine.

12. Procédé selon l'une quelconque des revendications précédentes, dans lequel le donneur d'amine est l'acide glutamique ou acide aspartique.

13. Procédé selon la revendication 12, dans lequel le donneur d'amine est l'acide aspartique.

14. Procédé selon l'une quelconque des revendications précédentes, dans lequel on utilise des cellules intactes contenant le micro-organisme.

15. Procédé selon l'une quelconque des revendications précédentes, dans lequel on ajoute dans la réaction de l'acide formique.

16. Procédé de préparation de la phénylalanine par transamination entre un donneur d'amine et l' acide phénylpyruvique en présence d'une aminotransférase obtenue à partir d'un micro-organisme qui a été génétiquement modifié pour produire en grande quantité l'aminotransférase, dans lequel la concentration du précurseur cétoacide est supérieure à 10g/l.

17. Procédé selon la revendication 16, dans lequel le donneur d'amine est l'acide glutamique ou l'acide aspartique.

18. Procédé selon la revendication 16 ou 17, dans lequel la réaction est conduite à une température dans la gamme de 45 à 70° C.

19. Procédé selon l'une quelconque des revendications 16 à 18, dans lequel la concentration du précurseur est de 40 à 50g/l,

20. Procédé selon l'une quelconque des revendications 16 à 19, dans lequel le gène codant pour l'aminotransférase est aspC, tyrB ou ilyE.

21. Procédé selon la revendication 20, dans lequel le micro-organisme est Escherichia coli ATCC 39501.

**Ansprüche**

1. Verfahren zur Herstellung einer Aminosäure durch Transaminierung zwischen einem Amin-Donor und einem Ketosäure-Vorläufer der Aminosäure in Gegenwart einer aus einem zur Überproduktion der Aminotransferase genetisch modifizierten Mikroorganismus erhaltenen Aminotransferase, und in dem die Transaminierung bei einer Temperatur durchgeführt wird, die gleich oder größer als 40 °C ist.

2. Verfahren nach Anspruch 1, dadurch gekennzeichnet, daß die Aminosäure eine aromatische Aminosäure ist.

3. Verfahren nach Anspruch 2, dadurch gekennzeichnet, daß die Aminosäure Phenylalamin, Tyrosin oder Hydroxymethoxyphenylalanin ist.

4. Verfahren nach Anspruch 3, dadurch gekennzeichnet, daß die Aminosäure Phenylalanin ist.

5. Verfahren nach Anspruch 4, dadurch gekennzeichnet, daß der Ketosäure-Vorläufer Phenylbrenztraubensäure ist und die Transaminierungsreaktion bei 40 °C bis 70 °C durchgeführt wird.

6. Verfahren nach Anspruch 5, dadurch gekennzeichnet, daß die Konzentration der Phenylbrenztraubensäure 30 bis 60 g/l ist.

7. Verfahren nach Anspruch 5 oder 6, dadurch gekennzeichnet, daß die Transaminierung bei 45 °C bis 60 °C durchgeführt wird.

8. Verfahren nach einem der Ansprüche 5 bis 7, dadurch gekennzeichnet, daß die Genkodierung für die Aminotransferase aspC, tyrB oder ilyE ist.

9. Verfahren nach Anspruch 8, dadurch gekennzeichnet, daß der Mikroorganismus Escherichia coli ist.

10. Verfahren nach Anspruch 9, dadurch gekennzeichnet, daß der Escherichia coli der Stamm ATCC 39501 ist.

11. Verfahren nach Anspruch 3, dadurch gekennzeichnet, daß die Aminosäure 3-Methoxy-4-hydroxyphenylalanin ist.

12. Verfahren nach einem der vorhergehenden Ansprüche, dadurch gekennzeichnet, daß der Amin-Donor Glutaminsäure oder Asparaginsäure ist.

13. Verfahren nach Anspruch 12, dadurch gekennzeichnet, daß der Amin-Donor Asparaginsäure ist.

14. Verfahren nach einem der vorhergehenden Ansprüche, dadurch gekennzeichnet, daß intakte Zellen, die den Mikroorganismus enthalten, verwendet werden.

15. Verfahren nach einem der vorhergehenden Ansprüche, dadurch gekennzeichnet, daß Ameisensäure zur Reaktion zugegeben wird.

16. Verfahren zur Herstellung von Phenylalanin durch Transaminierung zwischen ein Amin-Donor und Phenylbrenztraubensäure in Gegenwart einer aus einem zur Überproduktion der Aminotransferase genetisch modifizierten Mikroorganismus erhaltenen Aminotransferase, und in dem die Konzentration an Ketosäure-Vorläufer größer als 10 g/l ist.

17. Verfahren nach Anspruch 16, dadurch gekennzeichnet, daß der Amin-Donor Glutaminsäure oder Asparginsäure ist.

**18.** Verfahren nach Anspruch 16 oder 17, dadurch gekennzeichnet, daß die Reaktion bei einer Temperatur im Bereich von 45 °C bis 70 °C durchgeführt wird.

**19.** Verfahren nach einem der Ansprüche 16 bis 18, dadurch gekennzeichnet, daß die Vorläufer-Konzentration 40 bis 50 g/l ist.

**20.** Verfahren nach einem der Ansprüche 16 bis 19, dadurch gekennzeichnet, daß die Genkodierung für die Aminotransferase aspC, tyrB oder ilyE ist.

**21.** Verfahren nach Anspruch 20, dadurch gekennzeichnet, daß der Mikroorganismus Escherischia coli ATCC 39501 ist.